# EUROPEAN PATENT SPECIFICATION

(11) **EP 1 334 201 B1**
(45) Date of publication and mention of the grant of the patent: **07.07.2010**
(21) Application number: 01980716.3
(22) Date of filing: 02.11.2001
(51) Int. Cl.: C12N 15/86

(54) **DNA EXPRESSION VECTORS**
DNA-EXPRESSIONSVEKTOREN
VECTEURS D'EXPRESSION D'ADN

(30) Priority: 06.11.2000 GB 0027088
(43) Date of publication of application: 13.08.2003
(73) Proprietor: GLAXO GROUP LIMITED, Greenford, Middlesex UB6 0NN (GB)
(72) Inventor: CATCHPOLE, Ian, Richard, c/o GlaxoSmithKline, Stevenage, Hertfordshire SG1 2NY (GB); ELLIS, Jonathan, Henry, c/o GlaxoSmithKline, Stevenage, Hertfordshire SG1 2NY (GB); ERTL, Peter, Franz, c/o GlaxoSmithKline, Stevenage, Hertfordshire SG1 2NY (GB); RHODES, John, Richard, c/o GlaxoSmithKline, Stevenage, Hertfordshire SG1 2NY (GB)
(74) Representative: Easeman, Richard Lewis
(86) International application number: PCT/GB2001/004912
(87) International publication number: WO 2002/036792

(56) References cited:
- US-A- 5 385 839
- CHAPMAN B S ET AL: "EFFECT OF INTRON A FROM HUMAN CYTOMEGALOVIRUS (TOWNE) IMMEDIATE- EARLY GENE ON HETEROLOGOUS EXPRESSION IN MAMMALIAN CELLS" NUCLEIC ACIDS RESEARCH, OXFORD UNIVERSITY PRESS, SURREY, GB, vol. 19, no. 14, 25 July 1991 (1991-07-25), pages 3979-3986, XP000569788 ISSN: 0305-1048
- DAVIS M G ET AL: "TRANSFER AND EXPRESSION OF PLASMIDS CONTAINING HUMAN CYTOMEGALOVIRUS IMMEDIATE-EARLY GENE 1 PROMOTER-ENHANCER SEQUENCES IN EUKARYOTIC AND PROKARYOTIC CELLS" BIOTECHNOLOGY AND APPLIED BIOCHEMISTRY, vol. 10, no. 1, 1988, pages 6-12, XP001080299 ISSN: 0885-4513
- DATABASE EMBL [Online] EBI, Cambridge, UK.; 18 July 1996 (1996-07-18) WYBORSKI D.L.: "Cloning vector pCMVLacI from Lacswitch II System, complete sequence." Database accession no. U64448 XP002199154
- BOSHART M ET AL: "A VERY STRONG ENHANCER IS LOCATED UPSTREAM OF AN IMMEDIATE EARLY GENE OF HUMAN CYTOMEGALOVIRUS" CELL, vol. 41, no. 2, 1985, pages 521-530, XP001079009 ISSN: 0092-8674

## Description

### Field of the invention

The invention relates to the use of DNA vectors containing a transcription regulatory sequence derived from the human cytomegalovirus major immediate early gene in the manufacture of vaccine compositions for raising an immune response. In particular, the invention provides vectors containing a minimal promoter region and a fragment of the 5' untranslated region of the human cytomegalovirus major immediate early gene which includes exon 1 but not intron A.

### Background to the invention

It is known to use the promoter and upstream enhancer regions of the human cytomegalovirus major immediate early gene (abbreviated herein to HCMV IE1) to drive expression of recombinant proteins. For example, European patent number EP 0 323 997 B describes expression vectors incorporating the promoter, upstream enhancer and a functionally complete 5' untranslated region of the HCMV IE1 gene, including the first intron. In such constructs the 5' UTR of HCMV IE1 is linked directly to the coding region of a heterologous gene, replacing the natural 5' UTR of the heterologous gene. Inclusion of the full-length 5' UTR significantly enhances the levels of expression beyond that observed with the minimal HCMV IE1 promoter alone.

It is generally accepted that the enhanced expression observed in the presence of the complete HCMV IE1 5' UTR is attributable to the inclusion of the first intron (referred to as intron A or intron 1). The present inventors have now surprisingly observed that the use of a fragment of the 5' UTR including exon 1 but lacking the first intron results in enhanced expression over and above the expression levels achieved with the HCMV IE1 minimal promoter alone. Moreover, replacement of the natural intron A of HCMV IE1 with an heterologous intron also results in enhanced expression. The enhancement of expression by the use of exon 1 in the absence of intron A was entirely unexpected from prior knowledge of the behaviour of the minimal HCMV promoter and 5' UTR.

The natural 5' UTR of the HCMV IE1 gene is relatively large (1021 bases). The use of exon 1 in the absence of intron A has the potential to allow the size of the promoter/5' UTR to be minimised, whilst maintaining efficient expression of recombinant proteins. This will have utility in the DNA vaccine field, where it is advantageous to minimise the length of non-coding sequences included in a DNA vaccine construct, and also in plasmid vectors containing multiple expression cassettes where it will minimise the possibility of recombination through homologous sequences within the plasmid.

### Description of the invention

In a first aspect the invention provides use of a vector containing a DNA sequence comprising a promoter, a fragment of the 5' untranslated region of the HCMV IE1 gene including all of exon 1 but wherein no more than 50 consecutive bases of intron A are present, and a DNA sequence encoding a polypeptide operably linked to the promoter and the fragment of the 5' untranslated region of the HCMV IE1 gene, in the manufacture of a vaccine composition for raising an immune response against said polypeptide.

The invention is based on the observation that a fragment of the HCMV IE1 5' untranslated region which includes exon 1 but not intron A is capable of enhancing the level of expression from a basic promoter, such as the HCMV IE1 minimal promoter.

A promoter may be generally defined as a region of DNA which is capable of directing initiation of transcription by RNA polymerase. The promoter used in the present invention may be essentially any RNA polymerase II-dependent promoter.

In a preferred embodiment, the promoter may be the HCMV IE1 minimal promoter. An HCMV IE1 minimal promoter may be defined as a fragment of the HCMV IE1 promoter region which is capable of functioning as a promoter, driving transcription from the natural transcription start site. For example, a fragment of the HCMV IE1 gene comprising nucleotides -116 to +1, nucleotide +1 being the HCMV IE1 transcription start site, exhibits promoter activity.

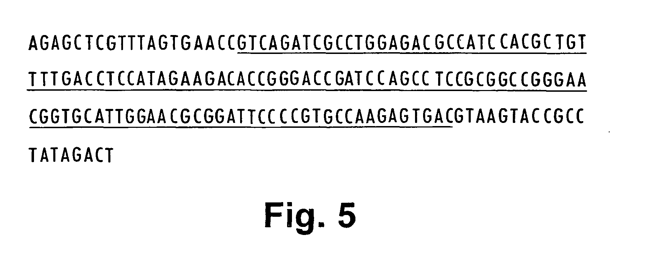

The fragment of the 5' untranslated region of HCMV IE1 will most preferably be positioned immediately 3' to (i.e. downstream of) the promoter, such that the HCMV 5' untranslated sequence will be included in transcripts which initiate at the transcription start site associated with the promoter. The nucleotide sequence of HCMV IE1 exon 1 from the Towne strain of HCMV is illustrated in the accompanying Figure 5. However, it is not intended for the term "an HCMV IE1 exon 1" to be limited to this precise sequence. This term also encompass minor variants, including exon 1 sequences from other strains of HCMV, such as AD169. The exon 1 from AD169 is between 514-634 of the sequence disclosed by A Krigg. A. et al Virus research 2, 107-121 (1985) and also variant sequences which exhibit base substitutions, insertions, additions and deletions. The skilled reader will appreciate that minor variation may be made to the exon 1 sequence without substantially affecting its ability to enhance expression from an associated promoter.

In one embodiment the vector may additionally comprise a heterologous intron, i.e. an intron other than intron A of the HCMV IE1 gene, positioned immediately downstream of exon 1 of HCMV IE1. The heterologous intron may replace the natural intron A in the HCMV IE1 5' UTR, in which case the untranslated part of HCMV IE1 exon 2 may be included immediately downstream of the heterologous intron. The heterologous intron may be synthetic or a naturally occurring intron other than intron A. The heterologous intron will be transcribed together with the fragment of the HCMV IE1 5' untranslated region, forming part of the 5' UTR of the resultant transcript. Intron A of AD169 can be located at position 635-1461 of the sequence disclosed by A Krigg. A. et al **supra**.

As illustrated in the accompanying examples, the inclusion of a heterologous intron may increase expression levels above that achieved using a promoter and exon 1 alone. Advantageously, the heterologous intron will be short (preferably less than 100 bases) in order to reduce the amount of non-coding sequence present in the vector. A suitable example is the first intron of the human CD68 gene which is 87 bases in length, but other heterologous introns may be used with equivalent effect.

The vector may further include restriction sites to allow for insertion of a heterologous coding sequence. The restriction sites will preferably be positioned downstream of the HCMV IE1 5' untranslated fragment, including any heterologous intron which may be included in the vector.

The vector may include one or more further transcription regulatory elements in addition to the promoter, such as enhancer elements. For example, vectors containing the minimal HCMV IE1 promoter may additionally include the HCMV IE1 enhancer element. Most preferably, the enhancer element will be positioned immediately upstream of the minimal HCMV IE1 promoter.

In a preferred embodiment, the vector may be a plasmid. A plasmid vector may further contain an origin of replication to allow autonomous replication within a prokaryotic host cell and a selective marker, such as an antibiotic resistance gene. The vector may also contain a pol II terminator to terminate transcription and a poly-adenylation signal for stabilization and processing of the 3' end of an mRNA transcribed from the promoter. Advantageously, one or more restriction sites may be included between the HCMV IE1 5' UTR sequence and the poly-adenylation signal to facilitate insertion of a heterologous coding sequence. Plasmid vectors according to the invention may be easily constructed from the component sequence elements using standard recombinant techniques well known in the art and described, for example, in F. M. Ausubel et al. (eds.), Current Protocols in Molecular Biology, John Wiley & Sons, Inc. (1994).

In a particularly preferred embodiment, the vector may be an expression vector for use in the expression of a recombinant polypeptide in a eukaryotic host cell or organism. In this embodiment the vector may further comprise a DNA sequence encoding a recombinant polypeptide operably linked to the HCMV IE1 minimal promoter and 5' UTR sequence. The term "operably linked" refers to an arrangement in which the polypeptide-encoding DNA sequence is positioned downstream of the promoter and 5' UTR such that transcription initiation at the transcription start site associated with the promoter results in transcription of an mRNA incorporating the HCMV IE1 5' UTR fragment (including any heterologous intron) and the sequence encoding the recombinant polypeptide.

The expression vector may contain a pol II terminator to terminate transcription and a poly-adenylation signal for stabilization and processing of the 3' end of an mRNA transcribed from the promoter. Suitable polyadenylation signals include mammalian polyadenylation signals such as, for example, the rabbit beta globin polyadenylation signal or the bovine growth hormone polyadenylation signal and also polyadenylation signals of viral origin, such as the SV40 late poly(A) region. The vector may further contain a selective marker which allows selection in eukaryotic host cells, for example a neomycin phosphotransferase marker. The expression vector may also contain one or more further expression cassettes to allow for expression of multiple recombinant polypeptides from a single vector. Most preferably, the expression vector will be a plasmid expression vector.

The DNA sequence encoding the recombinant polypeptide may be essentially any protein-encoding DNA sequence bounded by start and stop codons. This protein-encoding DNA sequence may include introns. In a particularly preferred embodiment the recombinant polypeptide may be an antigenic polypeptide or therapeutic protein.

In a preferred embodiment the antigen is capable of eliciting an immune response against a human pathogen, which antigen or antigenic composition is derived from HIV-1, (such as tat, nef, gp120 or gp160, gp40, p24, gag, env, vif, vpr, vpu, rev), human herpes viruses, such as gH, gL gM gB gC gK gE or gD or derivatives thereof or Immediate Early protein such as ICP27, ICP 47, IC P 4, ICP36 from HSV1 or HSV2, cytomegalovirus, especially Human, (such as gB or derivatives thereof), Epstein Barr virus (such as gp350 or derivatives thereof), Varicella Zoster Virus (such as gpI, II, III and IE63), or from a hepatitis virus such as hepatitis B virus (for example Hepatitis B Surface antigen or Hepatitis core antigen or pol), hepatitis C virus antigen and hepatitis E virus antigen, or from other viral pathogens, such as paramyxoviruses: Respiratory Syncytial virus (such as F and G proteins or derivatives thereof), or antigens from parainfluenza virus, measles virus, mumps virus, human papilloma viruses (for example HPV6, 11, 16, 18, eg L1, L2, E1, E2, E3, E4, E5, E6, E7), flaviviruses (e.g. Yellow Fever Virus, Dengue Virus, Tick-borne encephalitis virus, Japanese Encephalitis Virus) or Influenza virus cells, such as HA, NP, NA, or M proteins, or combinations thereof), or antigens derived from bacterial pathogens such as *Neisseria spp*, including *N*. *gonorrhea* and *N*. *meningitidis,* eg, transferrin-binding proteins, lactoferrin binding proteins, PilC, adhesins); *S*. *pyogenes* (for example M proteins or fragments thereof, C5A protease, S. *agalactiae, S*. *mutans*; *H. ducreyi; Moraxella spp, including M catarrhalis, also known as Branhamella* catarrhalis (for example high and low molecular weight adhesins and invasins); *Bordetella spp, including B. pertussis* (for example pertactin, pertussis toxin or derivatives thereof, filamenteous hemagglutinin, adenylate cyclase, fimbriae), *B. parapertussis and B. bronchiseptica; Mycobacterium spp., including M. tuberculosis* (for example ESAT6, Antigen 85A, -B or - C, MPT 44, MPT59, MPT45, HSP10,HSP65, HSP70, HSP 75, HSP90, PPD 19kDa [Rv3763], PPD 38kDa [Rv0934] ), *M*. *bovis, M. leprae, M. avium*, *M. paratuberculosis, M. smegmatis; Legionella spp,* including *L. pneumophila; Escherichia spp,* including *enterotoxic E. coli* (for example colonization factors, heat-labile toxin or derivatives thereof, heat-stable toxin or derivatives thereof), enterohemorragic *E*. *coli,* enteropathogenic *E. coli* (for example shiga toxin-like toxin or derivatives thereof); *Vibrio spp,* including *V. cholera* (for example cholera toxin or derivatives thereof); *Shigella spp,* including *S*. *sonnei, S. dysenteriae, S. flexnerii; Yersinia spp,* including Y. enterocolitica (for example a Yop protein) , *Y. pestis, Y. pseudotuberculosis; Campylobacter spp,* including *C*. *jejuni* (for example toxins, adhesins and invasins) and *C. coli; Salmonella spp,* including *S. typhi, S. paratyphi, S. choleraesuis, S. enteritidis; Listeria spp.,* including *L. monocytogenes; Helicobacter spp,* including *H*. pylori (for example urease, catalase, vacuolating toxin); *Pseudomonas spp,* including *P*. *aeruginosa; Staphylococcus spp.,* including *S. aureus, S. epidermidis; Enterococcus spp.,* including *E*. *faecalis, E. faecium; Clostridium spp.,* including *C*. tetani (for example tetanus toxin and derivative thereof), *C. botulinum* (for example botulinum toxin and derivative thereof), *C*. *difficile* (for example clostridium toxins A or B and derivatives thereof); *Bacillus spp.,* including *B. anthracis* (for example botulinum toxin and derivatives thereof); *Corynebacterium spp.,* including *C*. *diphtheriae* (for example diphtheria toxin and derivatives thereof); *Borrelia spp.,* including *B. burgdorferi* (for example OspA, OspC, DbpA, DbpB), *B. garinii* (for example OspA, OspC, DbpA, DbpB), *B. afzelii* (for example OspA, OspC, DbpA, DbpB), *B. andersonii* (for example OspA, OspC, DbpA, DbpB), *B. hermsii; Ehrlichia spp.,* including *E*. *equi* and the agent of the Human Granulocytic Ehrlichiosis; *Rickettsia spp,* including *R. rickettsii; Chlamydia spp*., *including C. trachomatis* (for example MOMP, heparin-binding proteins), *C. pneumoniae* (for example MOMP, heparin-binding proteins), *C. psittaci; Leptospira spp.,* including *L. interrogans; Treponema spp.,* including *T. pallidum* (for example the rare outer membrane proteins), *T. denticola, T. hyodysenteriae;* or derived from parasites such as *Plasmodium spp.,* including *P. falciparum; Toxoplasma spp.,* including *T. gondii (for example SAG2, SAG3, Tg34); Entamoeba spp.,* including *E. histolytica; Babesia spp.,* including *B. microti; Trypanosoma spp.,* including *T. cruzi; Giardia spp.,* including *G*. *lamblia; Leshmania spp.,* including *L. major; Pneumocystis spp.,* including *P. carinii; Trichomonas spp.,* including *T. vaginalis; Schisostoma spp.,* including *S. mansoni,* or derived from yeast such as *Candida spp.,* including *C*. *albicans; Cryptococcus spp.,* including *C*. *neoformans.*

Other preferred specific antigens for *M. tuberculosis* are for example Rv2557, Rv2558, RPFs: Rv0837c, Rv1884c, Rv2389c, Rv2450, Rev1009, aceA (Rv0467), PstS1, (Rv0932), SodA (Rv3846), Rv2031c 16kDal., Tb Ra12, Tb H9, Tb Ra35, Tb38-1, Erd 14, DPV, MTI, MSL, mTTC2 and hTCC1 (WO 99/51748). Proteins for *M. tuberculosis* also include fusion proteins and variants thereof where at least two, preferably three polypeptides of *M. tuberculosis* are fused into a larger protein. Preferred fusions include Ra12-TbH9-Ra35, Erd14-DPV-MTI, DPV-MTI-MSL, Erd14-DPV-MTI-MSL-mTCC2, Erd14-DPV-MTI-MSL, DPV-MTI-MSL-mTCC2, TbH9-DPV-MTI (WO 99/51748).

Most preferred antigens for Chlamydia include for example the High Molecular Weight Protein (HWMP) (WO 99/17741), ORF3 (EP 366 412), and putative membrane proteins (Pmps). Other Chlamydia antigens of the vaccine formulation can be selected from the group described in WO 99/28475.

Preferred bacterial vaccines comprise antigens derived from *Streptococcus spp,* including *S*. *pneumoniae* (PsaA, PspA, streptolysin, choline-binding proteins) and the protein antigen Pneumolysin (Biochem Biophys Acta, 1989, 67, 1007; Rubins et al., Microbial Pathogenesis, 25, 337-342), and mutant detoxified derivatives thereof (WO 90/06951; WO 99/03884). Other preferred bacterial vaccines comprise antigens derived from *Haemophilus spp., including H. influenzae type B* (for example PRP and conjugates thereof), *non typeable H. influenzae,* for example OMP26, high molecular weight adhesins, P5, P6, protein D and lipoprotein D, and fimbrin and fimbrin derived peptides (US 5,843,464) or multiple copy variants or fusion proteins thereof.

The antigens that may be used in the present invention may further comprise antigens derived from parasites that cause Malaria. For example, preferred antigens from *Plasmodia falciparum* include RTS,S and TRAP. RTS is a hybrid protein comprising substantially all the C-terminal portion of the circumsporozoite (CS) protein of *P.falciparum* linked via four amino acids of the preS2 portion of Hepatitis B surface antigen to the surface (S) antigen of hepatitis B virus. It's full structure is disclosed in the International Patent Application No. PCT/EP92/02591, published under Number WO 93/10152 claiming priority from UK patent application No.9124390.7. Other plasmodia antigens that are likely candidates to be components of a multistage Malaria vaccine are P. *faciparum* MSP1, AMA1, MSP3, EBA, GLURP, RAP1, RAP2, Sequestrin, PfEMP1, Pf332, LSA1, LSA3, STARP, SALSA, PfEXP1, Pfs25, Pfs28, PFS27/25, Pfs16, Pfs48/45, Pfs230 and their analogues in Plasmodium spp.

The invention contemplates the use of an antitumour antigen and will be useful for the immunotherapeutic treatment of cancers. For example, tumour rejection antigens such as those for prostrate, breast, colorectal, lung, pancreatic, renal or melanoma cancers. Exemplary antigens include MAGE 1, 3 and MAGE 4 or other MAGE antigens such as disclosed in WO99/40188, PRAME, BAGE, Lage (also known as NY Eos 1) SAGE and HAGE (WO 99/53061) or GAGE (Robbins and Kawakami, 1996, Current Opinions in Immunology 8, pps 628-636; Van den Eynde et al., International Journal of Clinical & Laboratory Research (submitted 1997); Correale et al. (1997), Journal of the National Cancer Institute 89, p293. Indeed these antigens are expressed in a wide range of tumour types such as melanoma, lung carcinoma, sarcoma and bladder carcinoma.

MAGE antigens for use in the present invention may be expressed as a fusion protein with an expression enhancer or an Immunological fusion partner. In particular, the Mage protein may be fused to Protein D from Heamophilus influenzae B. In particular, the fusion partner may comprise the first 1/3 of Protein D. Such constructs are disclosed in WO99/40188. Other examples of fusion proteins that may contain cancer specific epitopes include *bcr* / *abl* fusion proteins.

In a preferred embodiment prostate antigens are utilised, such as Prostate specific antigen (PSA), PAP, PSCA (PNAS 95(4) 1735 -1740 1998), PSMA or antigen known as Prostase.

Prostase is a prostate-specific serine protease (trypsin-like), 254 amino acid-long, with a conserved serine protease catalytic triad H-D-S and a amino-terminal pre-propeptide sequence, indicating a potential secretory function (P. Nelson, Lu Gan, C. Ferguson, P. Moss, R. Gelinas, L. Hood & K. Wand, "Molecular cloning and characterisation of prostase, an androgen-regulated serine protease with prostate restricted expression, In Proc. Natl. Acad. Sci. USA (1999) 96, 3114-3119). A putative glycosylation site has been described. The predicted structure is very similar to other known serine proteases, showing that the mature polypeptide folds into a single domain. The mature protein is 224 amino acids-long, with one A2 epitope shown to be naturally processed.

Prostase nucleotide sequence and deduced polypeptide sequence and homologs are disclosed in Ferguson, et al. (Proc. Natl. Acad. Sci. USA 1999, 96, 3114-3119) and in International Patent Applications No. WO 98/12302 (and also the corresponding granted patent US 5,955,306), WO 98/20117 (and also the corresponding granted patents US 5,840,871 and US 5,786,148) (prostate-specific kallikrein) and WO 00/04149 (P703P).

The present invention provides vectors that encode antigens comprising prostase protein fusions based on prostase protein and fragments and homologues thereof ("derivatives"). Such derivatives are suitable for use in therapeutic vaccine formulations which are suitable for the treatment of prostate tumours. Typically the fragment will contain at least 20, preferably 50, more preferably 100 contiguous amino acids as disclosed in the above referenced patent and patent applications.

A further preferred prostate antigen is known as P501S, sequence ID no 113 of WO98/37814. Immunogenic fragments and portions encoded by the gene thereof comprising at least 20, preferably 50, more preferably 100 contiguous amino acids as disclosed in the above referenced patent application, are contemplated. A particular fragment is PS108 (WO 98/50567).

Other prostate specific antigens are known from Wo98/37418, and WO/004149. Another is STEAP PNAS 96 14523 14528 7 -12 1999.

Other tumour associated antigens useful in the context of the present invention include: Plu -1 J Biol. Chem 274 (22) 15633 -15645, 1999, HASH -1, HasH-2, Cripto (Salomon et al Bioessays 199, 21 61 -70,US patent 5654140) Criptin US patent 5,981,215. Additionally, antigens particularly relevant for vaccines in the therapy of cancer also comprise tyrosinase and survivin.

The present invention is also useful in combination with breast cancer antigens such as Muc-1, Muc-2, EpCAM, her 2/ Neu, mammaglobin (US patent 5668267) or those disclosed in WO/00 52165, WO99/33869, WO99/19479, WO 98/45328. Her 2 neu antigens are disclosed inter alia, in US patent 5,801,005. Preferably the Her 2 neu comprises the entire extracellular domain (comprising approximately amino acid 1 -645) or fragmants thereof and at least an immunogenic portion of or the entire intracellular domain approximately the C terminal 580 amino acids. In particular, the intracellular portion should comprise the phosphorylation domain or fragments thereof. Such constructs are disclosed in WO00/44899.

The her 2 neu as used herein can be derived from rat, mouse or human.

The vaccine may also contain antigens associated with tumour-support mechanisms (e.g. angiogenesis, tumour invasion), for example tie 2, VEGF.

Vaccines of the present invention may also be used for the prophylaxis or therapy of chronic disorders in addition to allergy, cancer or infectious diseases. Such chronic disorders are diseases such as asthma, atherosclerosis, and Alzheimers and other auto-immune disorders. Vaccines for use as a contraceptive may also be considered.

Antigens relevant for the prophylaxis and the therapy of patients susceptible to or suffering from Alzheimer neurodegenerative disease are, in particular, the N terminal 39-43 amino acid fragment of the (β amyloid precursor protein and smaller fragments. This antigen is disclosed in the International Patent Application No. WO 99/27944 - (Athena Neurosciences).

Potential self-antigens that could be included as vaccines for auto-immune disorders or as a contraceptive vaccine include: cytokines, hormones, growth factors or extracellular proteins, more preferably a 4-helical cytokine, most preferably IL13. Cytokines include, for example, IL1, IL2, IL3, IL4, IL5, IL6, IL7, IL8, IL9, IL10, IL11, IL12, IL13, IL14, IL15, IL16, IL17, IL18, IL20, IL21, TNF, TGF, GMCSF, MCSF and OSM. 4-helical cytokines include IL2, IL3, IL4, IL5, IL13, GMCSF and MCSF. Hormones include, for example, luteinising hormone (LH), follicle stimulating hormone (FSH), chorionic gonadotropin (CG), VGF, GHrelin, agouti, agouti related protein and neuropeptide Y. Growth factors include, for example, VEGF.

The vaccines of the present invention are particularly suited for the immunotherapeutic treatment of diseases, such as chronic conditions and cancers, but also for the therapy of persistent infections. Accordingly the vaccines of the present invention are particularly suitable for the immunotherapy of infectious diseases, such as Tuberculosis (TB), HIV infections such as AIDS and Hepatitis B (HepB) virus infections.

In an embodiment of the invention the antigen is a polynucleotide and is administered/delivered as "naked" DNA, for example as described in Ulmer et al., Science 259:1745-1749, 1993 and reviewed by Cohen, Science 259:1691-1692, 1993. Here the DNA is formulated in a buffered saline solution. The uptake of naked DNA may be increased by coating the DNA onto biodegradable beads or naturally eliminated, which are efficiently transported into the cells or by using other well known transfection facilitating agents. DNA encoding the antigen may be administered in conjunction with a carrier such as, for example, liposomes. Typically such liposomes are cationic, for example imidazolium derivatives (WO95/14380), guanidine derivatives (WO95/14381), phosphatidyl choline derivatives (WO95/35301), piperazine derivatives (WO95/14651) and biguanide derivatives.

Vectors according to the invention which express antigenic peptides may be used as the basis of DNA vaccine compositions and immunotherapeutic compositions. In a similar manner, vectors that encode therapeutic proteins may be used as the basis of therapeutic compositions. Thus, the invention further provides for use of an expression vector as described herein which is suitable for expression of an antigenic peptide for the manufacture of an immunotherapeutic, vaccine or vaccine composition. A method of vaccinating a mammalian subject which comprises administering thereto an effective amount of such a vaccine or vaccine composition is described. Most preferably, expression vectors for use in DNA vaccines, vaccine compositions and immunotherapeutics will be plasmid vectors.

DNA vaccines may be administered in the form of "naked DNA", for example in a liquid formulation administered using a syringe or high pressure jet, or DNA formulated with liposomes or an irritant transfection enhancer, or by particle mediated DNA delivery (PMDD). All of these delivery systems are well known in the art. The vector may be introduced to a mammal for example by means of a viral vector delivery system.

The compositions described herein can be delivered by a number of routes such as intramuscularly, subcutaneously, intraperitonally or intravenously.

The vector is preferably delivered intradermally. In particular, the vector is delivered by means of a gene gun (particularly particle bombardment) administration techniques which involve coating the vector on to a bead (eg gold) which are then administered under high pressure into the epidermis; such as, for example, as described in Haynes et al, J Biotechnology 44: 37-42 (1996).

In one illustrative example, gas-driven particle acceleration can be achieved with devices such as those manufactured by Powderject Pharmaceuticals PLC (Oxford, UK) and Powderject Vaccines Inc. (Madison, WI), some examples of which are described in U.S. Patent Nos. 5,846,796; 6,010,478; 5,865,796; 5,584,807; and EP Patent No. 0500 799. This approach offers a needle-free delivery approach wherein a dry powder formulation of microscopic particles, such as polynucleotide, are accelerated to high speed within a helium gas jet generated by a hand held device, propelling the particles into a target tissue of interest, typically the skin. The particles are preferably gold beads of a 0.4 - 4.0 µm, more preferably 0.6 - 2.0 µm diameter and the DNA conjugate coated onto these and then encased in a cartridge or cassette for placing into the "gene gun".

Other devices and methods that may be useful for gas-driven needle-less injection of compositions described herein include those provided by Bioject, Inc. (Portland, OR), some examples of which are described in U.S. Patent Nos. 4,790,824; 5,064,913; 5,312,335; 5,383,851; 5,399,163; 5,520,639 and 5,993,412.

The vectors which comprise the nucleotide sequences encoding antigenic peptides are administered in such amount as will be prophylactically or therapeutically effective. The quantity to be administered, is generally in the range of one picogram to 1 milligram, preferably 1 picogram to 10 micrograms for particle-mediated delivery, and 10 micrograms to 1 milligram for other routes of nucleotide per dose. The exact quantity may vary considerably depending on the species and weight of the mammal being immunised and the route of administration.

It is possible for the immunogen component comprising the nucleotide sequence encoding the antigenic peptide, to be administered on a once off basis or to be administered repeatedly, for example, between 1 and 7 times, preferably between 1 and 4 times, at intervals between about 1 day and about 18 months. Once again, however, this treatment regime will be significantly varied depending upon the size and species of animal concerned, the disease which is being treated/protected against, the amount of nucleotide sequence administered, the route of administration, and other factors which would be apparent to a skilled veterinary or medical practitioner.

The vectors described herein may be utilised with immunostimulatory agents. Preferably the immunostimulatory agents are administered at the same time as the nucleic acid vector and are formulated together. Such immunostimulatory agents include, but this list is by no means exhaustive and does not preclude other agents: synthetic imidazoquinolines such as imiquimod [S-26308, R-837], (Harrison, et al. ' Reduction of recurrent HSV disease using imiquimod alone or combined with a glycoprotein vaccine', Vaccine 19: 1820-1826, (2001)); and resiquimod [S-28463, R-848] (Vasilakos, et al. 'Adjuvant activites of immune response modifier R-848: Comparison with CpG ODN', Cellular immunology 204: 64-74 (2000).), Schiff bases of carbonyls and amines that are constitutively expressed on antigen presenting cell and T-cell surfaces, such as tucaresol (Rhodes, J. et al. Therapeutic potentiation of the immune system by costimulatory Schiff-base-forming drugs', Nature 377: 71-75 (1995)), cytokine, chemokine and co-stimulatory molecules as either protein or peptide, this would include pro-inflammatory cytokines such as GM-CSF, IL-1 alpha, IL-1 beta, TGF- alpha and TGF - beta, Th1 inducers such as interferon gamma, IL-2, IL-12, IL-15 and IL-18, Th2 inducers such as IL-4, IL-5, IL-6, IL-10 and IL-13 and other chemokine and co-stimulatory genes such as MCP-1, MIP-1 alpha, MIP-1 beta, RANTES, TCA-3, CD80, CD86 and CD40L, other immunostimulatory targeting ligands such as CTLA-4 and L-selectin, apoptosis stimulating proteins and peptides such as Fas, (49), synthetic lipid based adjuvants, such as vaxfectin, (Reyes et al., 'Vaxfectin enhances antigen specific antibody titres and maintains Th1 type immune responses to plasmid DNA immunization', Vaccine 19: 3778-3786) squalene, alpha- tocopherol, polysorbate 80, DOPC and cholesterol, endotoxin, [LPS], Beutler, B., 'Endotoxin, 'Toll-like receptor 4, and the afferent limb of innate immunity', Current Opinion in Microbiology 3: 23-30 (2000)) ; CpG oligo- and di-nucleotides, Sato, Y. et al., 'Immunostimulatory DNA sequences necessary for effective intradermal gene immunization', Science 273 (5273): 352-354 (1996). Hemmi, H. et al., 'A Toll-like receptor recognizes bacterial DNA', Nature 408: 740-745, (2000) and other potential ligands that trigger Toll receptors to produce Th1-inducing cytokines, such as synthetic Mycobacterial lipoproteins, Mycobacterial protein p19, peptidoglycan, teichoic acid and lipid A.

Certain preferred adjuvants for eliciting a predominantly Th1-type response include, for example, a Lipid A derivative such as monophosphoryl lipid A, or preferably 3-de-O-acylated monophosphoryl lipid A. MPL^{®} adjuvants are available from Corixa Corporation (Seattle, WA; see, for example, US Patent Nos. 4,436,727; 4,877,611; 4,866,034 and 4,912,094). CpG-containing oligonucleotides (in which the CpG dinucleotide is unmethylated) also induce a predominantly Th1 response. Such oligonucleotides are well known and are described, for example, in WO 96/02555, WO 99/33488 and U.S. Patent Nos. 6,008,200 and 5,856,462. Immunostimulatory DNA sequences are also described, for example, by Sato et al., Science 273:352, 1996. Another preferred adjuvant comprises a saponin, such as Quil A, or derivatives thereof, including QS21 and QS7 (Aquila Biopharmaceuticals Inc., Framingham, MA); Escin; Digitonin; or *Gypsophila* or *Chenopodium quinoa* saponins.

The invention further provides host cells transformed or transfected with an expression vector according to the invention. The host cell may be essentially any eukaryotic cell, mammalian cells being most preferred.

The invention still further provides a process for the production of a recombinant polypeptide in a eukaryotic host cell, comprising introducing an expression vector according to the invention into the host cell and culturing the cell under conditions which allow for expression of the polypeptide.

The invention will be further understood with reference to the following experimental examples, together with the accompanying Figures, in which:

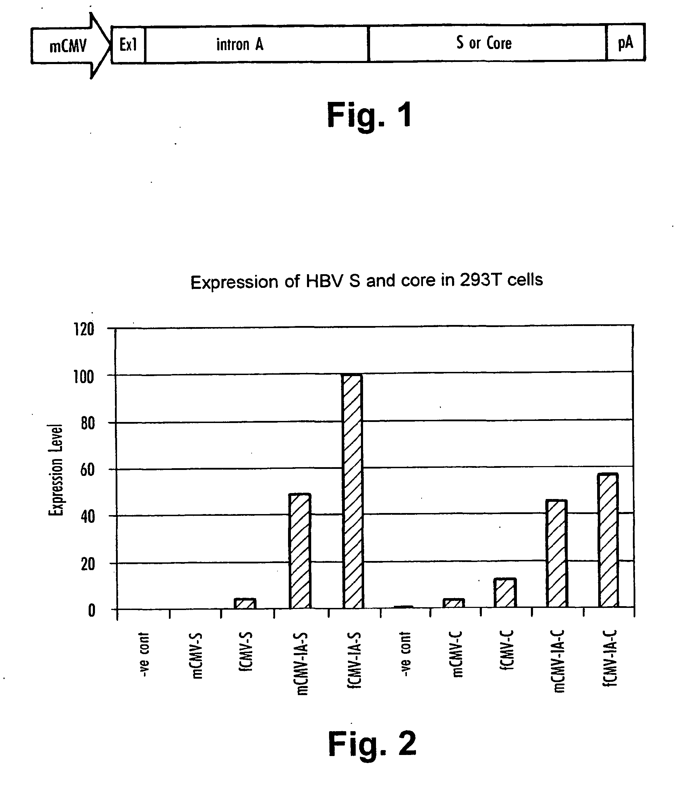

- Figure 1:: is a schematic representation of the standard expression cassette used for HBV core or S antigen expression. The S or Core gene was inserted into a plasmid 3' to a minimal HCMV IE1 promoter (mCMV) and intron A (nucleotides -116 to +958 relative to the transcription start), and 5' to a rabbit beta globin polyadenylation signal (pA). The plasmid backbone additionally contained a pUC19 origin of replication and a kanomycin selection marker.
- Figure 2:: is a graphical representation of the expression of HBV S and Core antigens from vectors with and without the IE1 5'UTR in 293T cells. The expression level of S is given in ng/ml of soluble S secreted into the culture medium. The level of core expression was determined by densitometry of a Western Blot, and is in arbitrary units. Key: mCMV=minimal CMV promoter; fCMV=full length CMV promoter; IA=Exon 1 and Intron A; S=Surface antigen; C=Core antigen.

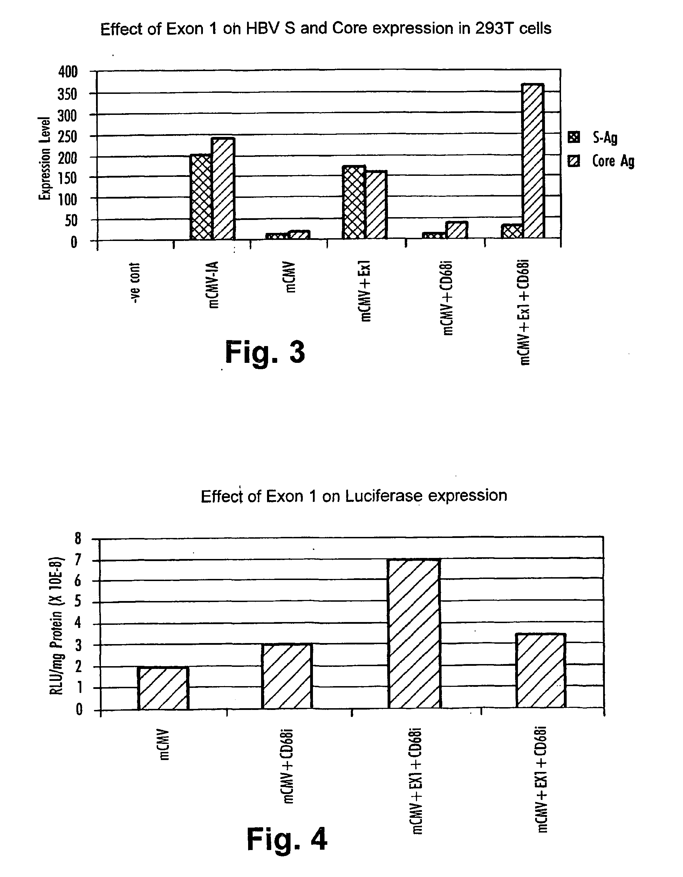

- Figure 3:: illustrates the effect of addition of Exon 1 in the absence of Intron A. The expression level of S antigen is given in ng/ml of soluble S antigen secreted into the culture medium. The level of core expression was determined by densitometry of a Western Blot, and is in arbitrary units. Key: mCMV=minimal CMV promoter; IA=Exon 1 and Intron A; EX1=Exon 1; CD68I=CD68 first intron.
- Figure 4:: illustrates the effect of Exon 1 on the level of expression of the Luciferase gene.
- Figure 5:: shows the sequence of a fragment of the major immediate early gene of the Towne strain of HCMV, including 19 bases of the promoter, the complete exon 1 and 20 bases of intron A. Exon 1 is underlined.

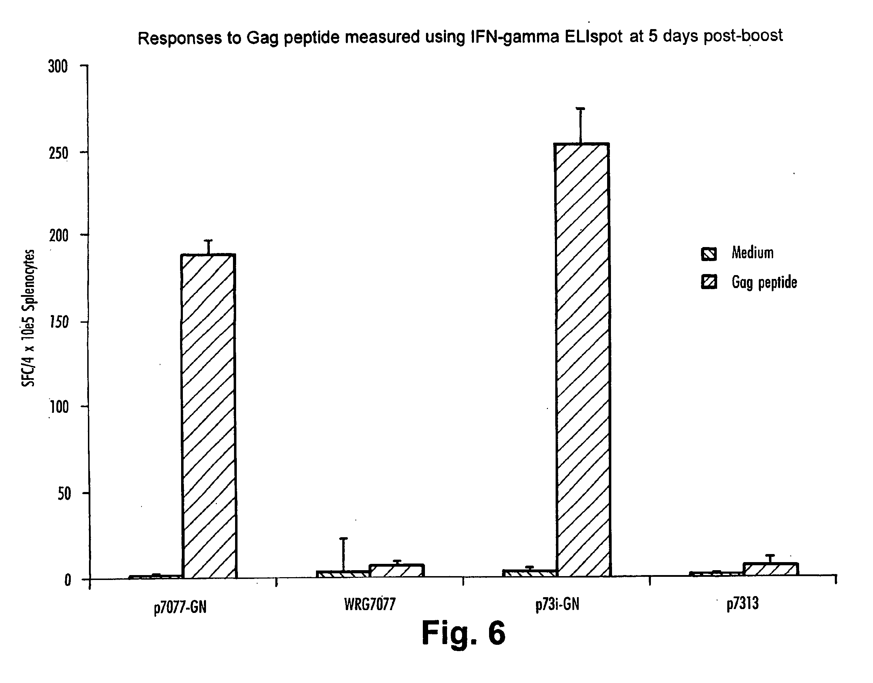

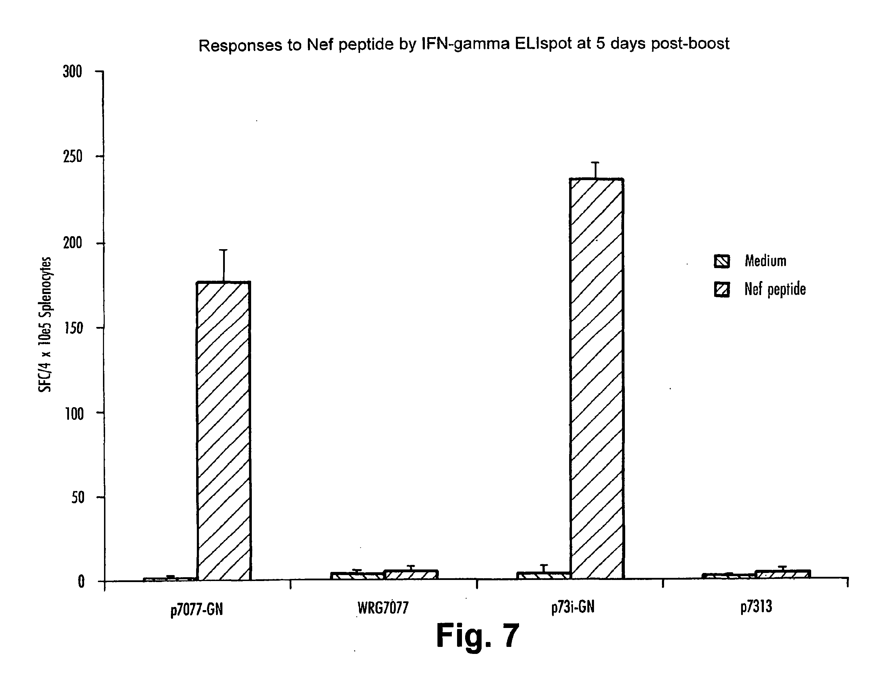

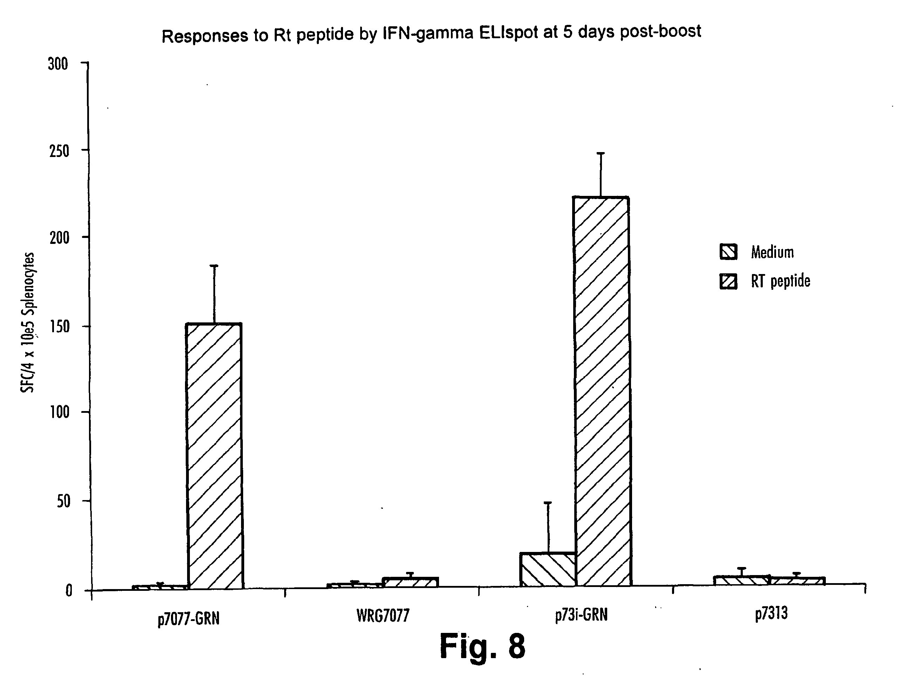

- Figures 6 - 8:: Shows the cellular response to the HIV antigens, NEF, RT and Gag generated by mice receiving DNA immunisation by means of particle mediated delivery. Mice either received DNA encoded antigen whose expression were driven by the HCMV IE promoter comprising Intron A and exon 1 (f cmv promoter) or HCMV IE promoter comprising exon 1, but in the absence of Intron A (I CMV).

### Example 1:

A number of plasmids were constructed to examine the efficiency of expression of the HBV S and Core antigens using different length HCMV IE1 promoters and 5' untranslated sequences (UTRs), usually incorporating an intron. A typical expression cassette is illustrated in Figure 1. It has been shown that expression of either antigen from a minimal CMV IE1 promoter gives very low levels of protein. Expression levels can be enhanced by increasing the promoter length to include the upstream enhancer region, or by addition of the natural 5' UTR of CMV IE1 (Figure 2). The natural 5' UTR sequence (nucleotides +1 to +958 relative to the transcription start site) includes the first untranslated exon, intron A and a few untranslated bases of the second exon.

The natural 5' UTR of CMV IE1 is relatively large (1021 bases). As convention suggests that the enhanced expression seen in the presence of the 5' UTR is attributable to the inclusion of the intron, experiments were designed to evaluate the effect of removing/substituting the intron, as follows:

A first set of constructs were made in which an alternative intron (the CD68 first intron of 87 bases) was cloned in place of the CMV 5' UTR. The CD68 intron was used either to replace the entire 5' UTR or placed 3' to exon 1 to replace intron A. When the entire 5' UTR was replaced by the CD68 intron very low levels of S or core antigen expression were observed. However, when exon 1 was retained in addition to the CD68 intron greatly enhanced expression of core was observed, though levels of S antigen expression were still relatively low (Figure 3).

A further construct was made in which the intron A sequence was removed entirely, leaving only exon 1 between the minimal CMV promoter and the recombinant gene. With this construct high levels of S antigen expression were observed. Expression of core antigen was also enhanced compared to levels from the minimal promoter alone, but not to the same levels observed in constructs containing either intron A or the CD68 intron in addition to exon 1 (Figure 3).

In further constructs, Exon 1 was also found to increase the level of expression of luciferase when placed between the minimal CMV promoter and the gene or upstream of CD68 exon 1 (Figure 4). This indicates that the enhancement of expression by inclusion of exon 1 in the absence of intron A is independent of the nature of the coding sequence being expressed.

Based on the results of these experiments it is concluded that inclusion of exon 1 in the absence of intron A enhances the level of expression of recombinant antigens from a minimal CMV promoter. This enhancement was not expected based on prior knowledge of the behaviour of the minimal CMV promoter and 5' UTR.

### Transfection methods and detection of expression products

293T cell monolayers (-2x10⁵ cells) in Corning CostarJ 24 well tissue culture dishes (Corning Incorporated, Corning NY 14831, USA)) were transfected with 1µg of DNA using 2.5µl of LipofectAMINEJ 2000 (Life Technologies, 3, Fountain Drive, Inchinnan Business Park Paisley, PA4 9RF) according to the manufacturer's protocol. After 24 hours the cells were resuspended into the culture medium by aspiration, and collected by centrifugation, and the cells were washed and resuspended in 250µl of phosphate buffered saline. The level of secreted S antigen was determined in the tissue culture supernatant by antibody capture, or alternatively the level of residual S-antigen in the cells was determined by immune staining with an anti-HBV-S antibody (DAKO M3506, Dako Corporation, Carpinteria, CA 93013, USA) detected with an FITC- conjugated anti-mouse antibody (Sigma F5897, Sigma-Aldrich Co. Ltd, Fancy Road, Pool, Dorset, BH12 4QH) followed by fluorescent microscopy using standard protocols (described in Antibodies, a Laboratory Manual (1998), Ed Harlow and David Lane (Ed) Cold Spring Harbor ISBN:0-87969-314-2 ). The level of core expression in the cells was determined by SDS Page and Western blot using an in-house guineapig antibody generated against purified HBV cores, and anti-guineapig horse radish peroxidase conjugate (DAKO P0141).

Quantitative S expression data was determined using an Origen M8 device. Surface antigen was measured in supernatants from transfected cells. Supernatants were mixed with two monoclonal antibodies to surface antigen, one labelled with biotin (C86312M from Biodesign International, 60 Industrial Park Road Saco, Maine 04072, USA) and the other with TAG (C86132M from Biodesign). After incubation, streptavidin coated beads were added to the samples. Surface antigen was quantitated by analysis of samples by Origen M8 Analyzer (IGEN Europe, Inc. Oxford BioBusiness Centre, Littlemore Park, Littlemore, Oxford OX4 2SS) United Kingdom, which detects specifically bound antibody.

### Example 2:

### Preparation of plasmid-coated 'gold slurry' for 'gene gun' DNA cartridges

Plasmid DNA (approximately 1µg/µl), eg. 100 ug, and 2µm gold particles, eg. 50 mg, (PowderJect), were suspended in 0.05M spermidine, eg. 100 ul, (Sigma). The DNA was precipitated on to the gold particles by addition of 1M CaCl₂, eg. 100ul (American Pharmaceutical Partners, Inc., USA). The DNA/gold complex was incubated for 10 minutes at room temperature, washed 3 times in absolute ethanol, eg. 3 x 1 ml, (previously dried on molecular sieve 3A (BDH)). Samples were resuspended in absolute ethanol containing 0.05mg/ml of polyvinylpyrrolidone (PVP, Sigma), and split into three equal aliquots in 1.5 ml microfuge tubes, (Eppendorf). The aliquots were for analysis of (a) 'gold slurry', (b) eluate- plasmid eluted from (a) and (c) for preparation of gold/ plasmid coated Tefzel cartridges for the 'gene gun', (see Example 3 below). For preparation of samples (a) and (b), the tubes containing plasmid DNA / 'gold slurry' in ethanol / PVP were spun for 2 minutes at top speed in an Eppendorf 5418 microfuge, the supernatant was removed and the 'gold slurry' dried for 10 minutes at room temperature. Sample (a) was resuspended to 0.5 - 1.0 ug / ul of plasmid DNA in TE pH 8.0, assuming approx. 50 % coating. For elution, sample (b) was resuspended to 0.5 - 1.0 ug / ul of plasmid DNA in TE pH 8.0 and incubated at 37°C for 30 minutes, shaking vigorously, and then spun for 2 minutes at top speed in an Eppendorf 5418 microfuge and the supernatant, eluate, was removed and stored at -20°C. The exact DNA concentration eluted was determined by spectrophotometric quantitation using a Genequant II (Pharmacia Biotech).

### Example 3:

### Preparations of cartridges for DNA immunisation

Preparation of cartridges for the Accell gene transfer device was as previously described (Eisenbraun et al DNA and Cell Biology, 1993 Vol 12 No 9 pp 791-797; Pertner et al). Briefly, plasmid DNA was coated onto 2 µm gold particles (DeGussa Corp., South Plainfield, N.J., USA) and loaded into Tefzel tubing, which was subsequently cut into 1.27 cm lengths to serve as cartridges and stored desiccated at 4°C until use. In a typical vaccination, each cartridge contained 0.5 mg gold coated with a total of 0.5 µg DNA/cartridge.

### Example 4: Immune Response to HIV antigen expressed under the control of HCMV It promoter in the absence of Intron A.

To examine whether exon 1 in the absence of Intron A enhances immune responses to HIV antigens delivered by Nucleic acid vaccination.

Mice (n=3/group) were vaccinated with antigens encoded by nucleic acid and located in two vectors. P7077 utilises the HCMV IE promoter including Intron A and exon 1 (fcmv promoter). P73I delivers the same antigen, but contains the HCMV IE promoter (icmv promoter) that is devoid of Intron A, but includes exon 1.

Plasmid was delivered to the shaved target site of abdominal skin of F1 (C3H x Balb/c) mice. Mice were given a primary immunisation of 2 x 0.5 µg DNA on day 0, boosted with 2 x 0.5 µg DNA on day 35 and cellular response were detected on day 40 using IFN - gamma Elispot.
- P73I - empty vector
- P7077 - empty vector
- P7077 GRN - (f CMV promoter) Gag, RT, Nef
- P73I GRN - (i CMV promoter) Gag, RT, Nef
- P7077 GN - (f CMV promoter)Gag, Nef
- P73I GN - (i CMV promoter) Gag, Nef

### Cytotoxic T Cell Responses

The cytotoxic T cell response was assessed by CD8+ T cell-restricted IFN-γ ELISPOT assay of splenocytes collected 5 days later. Mice were killed by cervical dislocation and spleens were collected into ice-cold PBS. Splenocytes were teased out into phosphate buffered saline (PBS) followed by lysis of red blood cells (1 minute in buffer consisting of 155mM NH₄Cl, 10 mM KHCO₃, 0.1mM EDTA). After two washes in PBS to remove particulate matter the single cell suspension was aliquoted into ELISPOT plates previously coated with capture IFN-γ antibody and stimulated with CD8- restricted cognate peptide (Gag, Nef or RT). After overnight culture, IFN-γ producing cells were visualised by application of anti-murine IFN-γ-biotin labelled antibody (Pharmingen) followed by streptavidin - conjugated alkaline phosphatase and quantitated using image analysis.

The results of this experiment are shown in figures 6, 7 and 8.

### Conclusion:

The inclusion of substantially all of exon 1 in the absence of Intron A enhances the level of expression of recombinant antigens from a minimal CMV promoter. The enhancement is independent of the antigen that is expressed. The vectors are useful in nucleic acid vaccination protocols and gene therapy protocols in providing enhanced expression of desired proteins **in vivo** and this expression is able to drive an immune response in vivo. Moreover, the vectors can increase the levels of recombinant proteins in culture.

## Claims

1. Use of a vector containing a DNA sequence comprising a promoter, a fragment of the 5' untranslated region of the human cytomegalovirus immediate-early (HCMV IE1) gene including all of exon 1 but wherein no more than 50 consecutive bases of intron A are present, and a DNA sequence encoding a polypeptide operably linked to the promoter and the fragment of the 5' untranslated region of the HCMV IE1 gene, in the manufacture of a vaccine composition for raising an immune response against said polypeptide.

2. Use of a vector as defined in claim 1 in the manufacture of an immunotherapeutic composition.

3. Use of a vector according to claim 1 or 2 wherein the promoter is an HCMV IE1 minimal promoter.

4. Use of a vector according to any one of claims 1 to 3 wherein the fragment of the 5' untranslated region of the HCMV IE1 gene is positioned immediately 3' to the promoter.

5. Use of a vector according to claim 4 which further comprises a heterologous intron sequence other than intron A of the HCMV IE1 gene positioned immediately downstream of HCMV IE1 exon 1 in the 5' untranslated region.

6. Use of a vector according to claim 4 or claim 5 which further comprises one or more restriction sites positioned downstream of the 5' untranslated region.

7. Use of a vector according to any one of claims 1 to 6 which is plasmid vector.

8. Use of a vector according to claim 1 wherein the polypeptide is an antigenic peptide.

9. Use of a vector as claimed in claim 1 to 8, said vaccine composition comprising a pharmaceutically acceptable adjuvant diluent, excipient or carrier.

10. Use of a vector according to claim 9 which carrier comprises a bead onto which the vector is coated.

11. A vector containing a DNA sequence comprising a promoter and a fragment of the 5' untranslated region of the HCMV IE1 gene including all of exon 1 but wherein no more than 50 consecutive bases of intron A are present and a DNA sequence encoding an antigenic peptide, for use as a vaccine, or immunotherapeutic or as a component of a vaccine composition or immunotherapeutic composition.

## Patentansprüche

1. Verwendung eines Vektors, enthaltend eine DNA-Sequenz, umfassend einen Promotor, ein Fragment der 5'-untranslatierten Region des menschlichen Cytomegalievirus Immediate-Early-Gens (HCMV IE1), einschliesslich des gesamten Exons 1, aber worin nicht mehr als 50 aufeinanderfolgende Basen des Introns A vorliegen, und eine DNA-Sequenz, die für ein Polypeptid codiert, die operabel mit dem Promotor und dem Fragment der 5'-untranslatierten Region des HCMV IE1-Gens verbunden ist, in der Herstellung einer Vakzinzusammensetzung zur Auslösung einer Immunantwort gegen dieses Polypeptid.

2. Verwendung eines Vektors, wie in Anspruch 1 definiert, in der Herstellung einer immunotherapeutischen Zusammensetzung.

3. Verwendung eines Vektors gemäss Anspruch 1 oder 2, worin der Promotor ein HCMV IE1-Minimalpromotor ist.

4. Verwendung eines Vektors gemäss einem der Ansprüche 1 bis 3, worin das Fragment der 5'-untranslatierten Region des HCMV IE1-Gens unmittelbar 3' zu dem Promotor positioniert ist.

5. Verwendung eines Vektors gemäss Anspruch 4, der ferner eine andere heterologe Intronsequenz als das Intron A des HCMV IE1-Gens umfasst, die unmittelbar downstream zum HCMV IE1 Exon 1 in der 5'-untranslatierten Region positioniert ist.

6. Verwendung eines Vektors gemäss Anspruch 4 oder Anspruch 5, der ferner eine oder mehrere Restriktionsstellen umfasst, die downstream zur 5'-untranslatierten Region positioniert sind.

7. Verwendung eines Vektors gemäss einem der Ansprüche 1 bis 6, der ein Plasmidvektor ist.

8. Verwendung eines Vektors gemäss Anspruch 1, worin das Polypeptid ein antigenes Peptid ist.

9. Verwendung eines Vektors gemäss den Ansprüchen 1 bis 8, wobei die Vakzinzusammensetzung ein(einen) pharmazeutisch akzeptable(s/n) Adjuvans, Verdünnungsmittel, Hilfsstoff oder Träger umfasst.

10. Verwendung eines Vektors gemäss Anspruch 9, wobei der Träger ein Bead umfasst, worauf der Vektor aufgetragen ist.

11. Vektor, enthaltend eine DNA-Sequenz, umfassend einen Promotor und ein Fragment der 5'-untranslatierten Region des HCMV IE1-Gens, einschliesslich des gesamten Exons 1, aber worin nicht mehr als 50 aufeinanderfolgende Basen des Introns A vorliegen, und eine DNA-Sequenz, die für ein antigenes Peptid codiert, zur Verwendung als Vakzin oder Immunotherapeutikum oder als ein Bestandteil einer Vakzinzusammensetzung oder einer immunotherapeutischen Zusammensetzung.

## Revendications

1. Utilisation d'un vecteur contenant une séquence d'ADN comprenant un promoteur, un fragment de la région non traduite en 5' du gène immédiat précoce du cytomégalovirus humain (HCMV IE1) y compris la totalité de l'exon 1 mais dans laquelle pas plus de 50 bases consécutives de l'intron A sont présentes, et une séquence d'ADN codant pour un polypeptide liée de manière fonctionnelle au promoteur et au fragment de la région non traduite en 5' du gène HCMV IE1, dans la fabrication d'une composition vaccinale destinée à soulever une réponse immunitaire contre ledit polypeptide.

2. Utilisation d'un vecteur tel que défini dans la revendication 1, dans la fabrication d'une composition immunothérapeutique.

3. Utilisation d'un vecteur selon la revendication 1 ou 2, où le promoteur est un promoteur minimal du HCMV IE1.

4. Utilisation d'un vecteur selon l'une quelconque des revendications 1 à 3, où le fragment de la région non traduite en 5' du gène HCMV IE1 est positionné immédiatement en 3' par rapport au promoteur.

5. Utilisation d'un vecteur selon la revendication 4, qui comprend en outre une séquence d'intron hétérologue autre que l'intron A du gène HCMV IE1 positionnée immédiatement en aval de l'exon 1 du HCMV IE1 dans la région non traduite en 5'.

6. Utilisation d'un vecteur selon la revendication 4 ou la revendication 5, qui comprend en outre un ou plusieurs sites de restriction positionnés en aval de la région non traduite en 5'.

7. Utilisation d'un vecteur selon l'une quelconque des revendications 1 à 6, qui est un vecteur plasmidique.

8. Utilisation d'un vecteur selon la revendication 1, où le polypeptide est un peptide antigénique.

9. Utilisation d'un vecteur tel que revendiqué dans la revendication 1 à 8, ladite composition vaccinale comprenant un adjuvant, un diluant, un excipient ou un support pharmaceutiquement acceptable.

10. Utilisation d'un vecteur selon la revendication 9, dont le support comprend une bille sur laquelle le vecteur est déposé.

11. Vecteur contenant une séquence d'ADN comprenant un promoteur et un fragment de la région non traduite en 5' du gène HCMV IE1 y compris la totalité de l'exon 1 mais dans laquelle pas plus de 50 bases consécutives de l'intron A sont présentes, et une séquence d'ADN codant pour un peptide antigénique, en vue d'une utilisation en tant que vaccin ou agent immunothérapeutique ou en tant que composant d'une composition vaccinale ou d'une composition immunothérapeutique.
